# EUROPEAN PATENT SPECIFICATION

(11) **EP 1 334 721 B1**
(45) Date of publication and mention of the grant of the patent: **22.04.2009**
(21) Application number: 01976818.3
(22) Date of filing: 23.10.2001
(51) Int. Cl.: A61K 31/198, A61K 9/20, A61K 9/28, A61P 3/10

(54) **NATEGLINIDE-CONTAINING HYDROPHILIC DRUG PREPARATIONS**
NATEGLINID ENTHALTENDE HYDROPHILE ARZNEIMITTELPRÄPARATE
PREPARATIONS DE MEDICAMENT CONTENANT DU NATEGLINIDE

(30) Priority: 24.10.2000 JP 2000324374
(43) Date of publication of application: 13.08.2003
(73) Proprietor: Ajinomoto Co., Inc., Tokyo 104-0031 (JP)
(72) Inventor: NINOMIYA, Nobutaka, c/o Ajinomoto Co., Inc., Kawasaki-Shi, Kanagawa 210-0801 (JP); MAKINO, Chisato, c/o Ajinomoto Co., Inc., Kawasaki-Shi, Kanagawa 210-0801 (JP); YABUKI, Akira, c/o Ajinomoto Co., Inc., Kawasaki-Shi, Kanagawa 210-0801 (JP)
(74) Representative: Nicholls, Kathryn Margaret
(86) International application number: PCT/JP2001/009292
(87) International publication number: WO 2002/040010

(56) References cited:
- EP-A1- 0 965 339
- EP-A2- 0 526 171
- JP-A- 6 183 955
- JP-A- 7 324 101

## Description

### Background of the Invention

The present invention relates to a preparation containing nateglinide as an effective ingredient which is useful as a blood glucose depressant. More specifically, the present invention relates to a hydrophilic pharmaceutical preparation containing B-type crystals of nateglinide

It is known that nateglinide [compound name: N-(trans -4- isopropyl cyclohexane carbonyl )-D-phenylalanine] exhibits an excellent blood glucose-lowering effect by oral administration and is useful as a therapeutic agent for diabetes (Japanese Patent Publication No. Hei 4-15221). In order to express the potential medical properties of nateglinide characterized by the quick action and short duration, this drug should be of immediate-release property. However, since nateglinide is a poorly water- soluble drug and thus a preparation having sufficient rapid-release property can not be obtained by conventional formulating methods, there have been proposed preparations
wherein a low-substituted hydroxypropyl cellulose is contained (Japanese Patent Un-examined Publication No. Hei 10-194969, equivalent to EP-A-0965339).

On the other hand, it is known that nateglinide has crystal polymorphs, and among them, H-type crystals are of the most stable crystal form (Japanese Patent No. 2508949). Although B-type crystals are inferior to H-type crystals in terms of stability, the former is superior to the latter in terms of the easiness of the preparation thereof, and hence there is a possibility in that said B-type crystals are actually employed.

In this connection, a preparation using H-type crystals according to the method described in Japanese Patent Un-examined Publication No. Hei 10-194969 has sufficient immediate-release properties, but when a preparation was prepared using B-type crystals, i.e., semi-stable type crystals, there could not be obtained a preparation having immediate-release properties at the same level as those of the preparation containing H-type crystals. Moreover, when the formulations were prepared with B-type crystals and adding a variety of excipients thereto, there occurred problems that powdered materials deposited onto apparatuses and the like for mixing and molding them depending on the types of additives, and therefore it took a long time to wash the apparatuses after operating.

### Disclosure of the Invention

An object of the present invention is to provide a preparation having sufficient immediate-release and high- dissolution rate, using B-type crystals of nateglinide.

Another object on the present invention is to provide methods by which a preparation having sufficient immediate-release and high-dissolution properties can be easily prepared using B-type crystals of nateglinide.

The present inventors have found that when each tablets having flat and smooth surface were prepared using each of nateglinide B-type crystals and nateglinide H-type crystals and the contact angle was determined by dropping water onto the respective surface of these tablets, the contact angle of the surface of tablets containing B-type crystals to water is 95 degree, and that of the surface of tablets containing H-type crystals to water is 85 degree, revealing that B-type crystals are more hydrophobic than H-type crystals. On the basis of this finding, the inventors have found that when various methods for formulating a preparation using B-type crystals of nateglinide were studied, there can be obtained a preparation having sufficient rapid-release and high-dissolution properties using B-type crystals of nateglinide, by preparing a preparation in which the contact angle of the surface thereof to water becomes 100 degrees or less, and the present invention has been completed.

Thus, the present invention provides a hydrophilic pharmaceutical preparation containing nateglinide B-type crystals as active ingredient and one or more further components which comprise a hydrophilic substance, the contact angle of the surface of said preparation to water being 100 degrees or less.

Further, the present invention provides methods for preparing a nateglinide-containing hydrophilic pharmaceutical preparation comprising formulating the preparation by adding hydrophilic substance(s) to formulation starting materials containing nateglinide B-type crystals as an effective ingredient so that the contact angle of the surface of said preparation to water becomes 100 degrees or less.

### Brief Description of Drawings

Figure 1 shows the profile of nateglinide concentration in plasma when nateglinide tablets are administrated to beagle dogs at 5 minutes before feeding: Mean ± SE; n = 3.
Figure 2 shows the profile of blood glucose level when nateglinide tablets are administrated to beagle dogs at 5 minutes before feeding: Mean ± SE; n = 3.
Figure 3 shows DSC pattern of tablets according to Example 1 before storing in aluminum pack at 40°C and 75% RH for 6 months.
Figure 4 shows DSC pattern of tablets according to Example 1 after storing in aluminum pack at 40°C and 75% RH for 6 months.
Figure 5 shows DSC pattern of tablets according to Example 2 before storing in aluminum pack at 40°C and 75% RH for 6 months.
Figure 6 shows DSC pattern of tablets according to Example 2 after storing in aluminum pack at 40°C and 75% RH for 6 months.
Figure 7 shows DSC pattern of tablets according to Example 3 before storing in aluminum pack at 40°C and 75% RH for 6 months.
Figure 8 shows DSC pattern of tablets according to Example 3 after storing in aluminum pack at 40°C and 75% RH for 6 months.

### Best mode for Carrying out the Invention

The preparation according to the present invention is one obtained by formulating nateglinide B-type crystals so that the contact angle of the surface of the obtained preparation composition to water becomes 100 degrees or less. In the case of a preparation employing nateglinide B-type crystals, when said contact angle is 100 degrees or less, there can be formulated a nateglinide B-type crystal-containing preparation having sufficient immediate-release and high-dissolution properties. Preferably, when said contact angle is 100 degree or less, more preferably 90 degree or less, a pharmaceutical preparation having more immediate-release property and higher dissolution properties can be obtained.

Furthermore, it may be better that said contact angle is 30 degree or higher in practical view point.

The term "contact angle to water" as employed herein means an angle
wherein drops of water to be deposited to the surface of preparation compositions such as tablets and the like contact with said surface, which can be used as an indication of hydrophilic nature. For example, such contact angle is measured by forming 1µL of droplet of pure water (MILLI-Q; MILLIPORE Co.) on the tip of needle (SNSO 52/026; made from stainless steel, inner diameter of 0.26 mm, outer diameter of 0.52 mm; HAMILTON Co.) and evaluating the contact angle at 60 m sec. after the droplet arrives onto the surface of tablet, using a contact angle measuring device (OCA-15 type; Data Physics Co.). When the surface of tablet has curvature, such contact angle is measured by correcting this curvature to straight line at the time when analysis is conducted. Usually, the contact angle is usually measured at room temperature.

As for preparations of which forms are not tablets, contact angles can be measured after molding powders, granules, semisolids and the like contained in such preparation. For example, in the case of capsules, the contact angle thereof can be determined by removing the contents of capsules and molding 200 mg of the contents into a tablet having diameter of 8 mm and thickness of 3.50 mm and having flat and smooth surface.

B-type nateglinide crystals employed in the invention can be obtained by dissolving nateglinide synthesized by various methods as described in Japanese Patent Publication No. Hei 4-15221 and Japanese Patent No. 2508949 in ethanol/water or acetone/water, cooling the resulting solution to 5°C to precipitate crystals, separating and drying the resulting crystals. The thus-obtained B-type crystals have a melting point of 129-130°C, and the X-ray diffraction pattern, infrared absorption spectrum and DSC chart of the crystal powders are described in the above-mentioned Japanese Patent documents.

In the pharmaceutical preparation containing nateglinide B-type crystals as an effective ingredient, the aim of preparing such preparation having surface contact angle to water of 100 degrees or less is achieved, by adding hydrophilic substance(s) to the composition.

The above "hydrophilic substance" is meant by pharmaceutically allowable material such as organic materials having hydrophilic group(s) or inorganic salts that possess large interaction and high affinity with water. Examples of the hydrophilic groups include a hydroxyl group, carboxyl group, amino group, pyrrolidone group and the like.

The hydrophilic substance in the invention is one having inorganic / organic ratio of 0.3 or more, preferably 0.7 or more, i.e., inorganic/organic ratio defined in the organic basic concept as described in Yoshio Kouda, "Organic Basic Concept - Fundament and Application - (Yuuki Gainennzu Kiso to Ouyou)" Sankyo Publisher (1984); Fujita and Masami Akatsuka, "Systematic Organic Qualitative Analysis (Keitouteki Yuuki Teisei Bunnseki) (Mixture Edition)", Fuma Shobou (1974); Yoshihiko Kuroki, "Dyeing Theoretical Chemistry (Sensyoku Riron Kagaku)", Maki Shoten (1966); Mitsuhiko Tobita and Yasuzou Uchida, "Fine Chemicals", Maruzen (1982); Hiroo Inoue, Satoshi Uehara and Mamoru Nango, "Methods for Separating Organic Compounds (Yuukikagoubutu Bunrihou)", Shoukabou (1990); and the like.

Specific examples include hydrophilic polymers having molecular weight of 500 or more such as polyvinyl pyrrolidone and derivatives thereof, polyvinyl alcohol and derivatives thereof, polysaccharide derivatives, polyether derivatives, and the like; anionic surfactants such as carboxyl acids, sulfuric esters, phosphoric esters and the like; nonionic surfactants such as ethers, esters and the like; sugars such as oligosaccharides, monosaccharides and the like; sugar alcohols such as mannitol, xylitol, erythritol, sorbitol, trehalose, maltitol and the like; and salts. Among them, hydrophilic polymers, anionic surfactants, nonionic surfactants and sugar alcohols are preferred, and in particular, mannitol, polyvinyl pyrrolidone, lactose (particularly, monohydrate), sodium lauryl sulfate, polysorbate and the like are preferred. Also, a combination of hydrophilic polymers and sugar alcohols is preferred.

Formulating amounts of these hydrophilic substances to nateglinide B-type crystals varydepending on the type of hydrophilic substances. For example, in the case of hydrophilic polymers, they can be employed in amounts of 1.2-fold or more, preferably 1.5-fold or more, more preferably 2-fold or more to the contents of nateglinide B-type crystals (weight base). Further, it is preferable to use such polymers in amounts of 100-fold or less in practical view point. In the case of surfactants, the amounts thereof can be adjusted to 1% or more to the contents of nateglinide. In this case, it is also preferable to use such surfactants in amounts of 20-fold or less in practical view point.

In addition to adding of said hydrophilic substance thereto, in order to achieve the object of the invention, the preparation composition may be film-coated or sugar-coated, whereby preparing nateglinide-containing hydrophilic pharmaceutical preparations having 100 degrees or less of contact angle to water, as film- or sugar-coated tablets.

When the nateglinide-containing hydrophilic pharmaceutical preparations are film-coated, hydrophilic polymers such as polyvinyl pyrrolidone derivatives, polyvinyl alcohol derivatives, polysaccharide derivatives, polyether derivatives and the like; or surfactants can be used as the film coating materials. In this case, it is preferable to use polyethylene glycol (molecular weight 1000-20000), hydroxypropylmetylcellulose 2910, polyvinyl pyrrolidone K90, polysorbate 80 and the like. Also, when the nateglinide-containing hydrophilic pharmaceutical preparations are sugar-coated tablets, oligosaccharides, monosaccharides and sugar alcohols can be used. The proportions of film coating materials used in film coat or sugar used as sugar-coating material vary depending on the type of film coating materials or sugars, but the proportions will be 30% or less to the weight of preparations to be coated.

In cases of film coated tablets or sugar coated tablets, it is preferable that the tablets to be coated also contain the hydrophilic substance(s) and have 100 degrees or less of contact angle to water.

The nateglinide-containing hydrophilic pharmaceutical preparations according to the invention may additionally comprise excipients and/or diluents and the like. Specific examples include light silicic anhydride, magnesium stearate, talc, titanium oxide, hydroxypropylcellulose, microcrystalline cellulose, calcium hydrogen phosphate, corn starch and the like.

The thus-prepared nateglinide B-type crystal containing preparations are pharmaceutical ones that show excellent immediate-release dissolution properties and can fully exhibit the characteristic of the quick action and short duration type of nateglinide preparations. Further, when the pharmaceutical preparations having the above properties according to the invention are prepared, addition of hydrophilic substance(s) that makes it possible to prepare such preparations having contact angle of 111 degree or 100 degrees or less, preferably 90 degrees or less to water,
into formulating starting materials containing nateglinide B-type crystals, can greatly improve the workability because raw compositions and the like deposit less onto the preparing apparatuses, and hence the washing procedure is easily conducted.

The following Examples and Comparative Examples will further illustrate the invention. These Examples will illustrate preferable embodiments according to the invention, which by no means limit the invention.

### Example 1

After sufficiently mixing 375 g of nateglinide B-type crystals, 262.5 g of mannitol, 75 g of light silicic anhydride and 750 g of crospovidone using a high speed agitation granulator (High Speed Mixer 10JD type; Freund Sangyo K.K.), 15 g of hydroxypropylcellulose dissolved in 1170 g of purified water was added thereto, and then high speed agitation granulation was carried out. The resulting granules were size-adjusted and dried, then 22.5 g of magnesium stearate was added, and compacted into 1500g of tablets having diameter of 7 mm and weight of 120 mg which contained 30 mg of nateglinide B-type crystals. Onto these tablets, 235 g of coating solution consisting of 8 g of hydroxypropylmethylcellulose, 1.5 g of polyethylene glycol 6000, 2.4 g of talc, 0.5 g of titanium oxide and 87.6 g of purified water was sprayed to obtain 1529 g of coated tablets. The contact angle of the resulting coated tablets was determined, and the contact angle was 87 degree. The dissolution properties in the solution No.2 employed in the Disintegration Test under the Japanese Pharmacopoeia was evaluated according to Japanese Pharmacopoeia Paddle Method (test liquid 500 ml: 50 revolutions/min.), and 100% of dissolution rate was observed in 30 minutes.

### Comparative Example 1

After sufficiently mixing 375 g of nateglinide B-type crystals, 637.5 g of lactose monohydrate and 450 g of low substituted hydroxypropyl cellulose (LH-31; Shinetsu Kagaku Kougyo K.K.) using a high speed agitation granulator (High Speed Mixer 10JD type; Freund Sangyo K.K.), 15 g of hydroxypropylcellulose dissolved in 1020 g of purified water was added thereto, and then high speed agitation granulation was carried out. The resulting granules were size-adjusted and dried, then 22.5 g of magnesium stearate was added, and compacted to obtain 1500 g of tablets having diameter of 7 mm and weight of 120 mg which contained 30 mg of nateglinide B-type crystals. The contact angle of the resulting tablets was determined, and the contact angle was 111 degree. The dissolution properties in the solution No.2 employed in the Disintegration Test under the Japanese Pharmacopoeia was evaluated according to Japanese Pharmacopoeia Paddle Method (test liquid 500 ml: 50 revolutions/min.), and 76% of dissolution rate was observed in 30 minutes.

### Example 2

Onto 1500 g of tablets obtained according to Comparative Example 1, 235 g of coating solution consisting of 8 g of hydroxypropylmethylcellulose, 1.5 g of polyethylene glycol 6000, 2.4 g of talc, 0.5 g of titanium oxide and 87.6 g of purified water was sprayed to obtain 1529 g of coated tablets. The contact angle of the resulting coated tablets was determined, and the contact angle was 76 degree. The dissolution property in the solution No.2 employed in the Disintegration Test under the Japanese Pharmacopoeia was evaluated according to Japanese Pharmacopoeia Paddle Method (test liquid 500 ml: 50 revolutions/min.), and 86% of dissolution rate was observed in 30 minutes.

### Example 3

After mixing 250 g of nateglinide B-type crystals, 225 g of microcrystalline cellulose, 500 g of crospovidone and 10 g of hydroxypropylcellulose using a V-type mixer, dry compression granulation using roller compactor (WP-90; Turbo Kougyo K.K.) and size adjustment were carried out to form granules of 850 µm or less. To these granules, 15 g of magnesium stearate was added, and compacted to obtain 1000 g of tablets having 120 mg weight (nateglinide: 30 mg). Onto these tablets, a coating solution consisting of 8 g of hydroxypropylmethylcellulose, 1.5 g of polyethylene glycol 6000, 2.4 g of talc, 0.5 g of titanium oxide and 87.6 g of purified water was sprayed so that 1.5 mg of hydroxypropylmethylcellulose was applied onto one tablet to obtain coated tablets. The contact angle of the coated tablets was 75 degree. The dissolution property in the solution No.2 employed in the Disintegration Test under the Japanese Pharmacopoeia was evaluated according to Japanese Pharmacopoeia Paddle Method (test liquid 500 ml: 50 revolutions/min.), and 99% of dissolution was observed in 30 minutes.

### Comparative Example 2

After sufficiently mixing 375 g of nateglinide B-type crystals, 780.8 g of lactose, 321.8 g of cornstarch and 15 g of hydroxypropylcellulose using a high speed agitation granulator (High Speed Mixer 10JD type; Furointo Sangyou K.K.), 310 g of purified water was added thereto, and then high speed agitation granulation was carried out. The resulting granules were size-adjusted and dried, then, 22.7 g of magnesium stearate was added, and compacted to obtain 1515 g of tablets having of diameter of 7 mm and weight of 121 mg which contained 30 mg of nateglinide B-type crystals. The contact angle of the resulting tablets was determined, and the contact angle was 115 degree. The dissolution property in the solution No.2 employed in the Disintegration Test under the Japanese Pharmacopoeia was evaluated according to Japanese Pharmacopoeia Paddle Method (test liquid 500 ml: 50 revolutions/min.), and less than 75% of dissolution rate was observed in 30 minutes.

### Experimental Example 1

After the high speed agitation granulation was carried out in Example 1,Comparative Example 1 and Comparative Example 2, the washability of the manufacturing machine (High Speed Mixer 10JD type; Freund Sangyo K.K.) was compared. After recovering the granules from the manufacturing machine, the easiness of washing out the deposits from the wall surface of the manufacturing machine was compared when flowing water was applied to the deposits.. The results obtained according to the following three-level evaluation are shown in Table 1 wherein Ⓞ deposits were well washed out; O deposits were washed out; × deposits were hardly washed out

**Table 1**

| | Example 1 | Comparative Example 1 | Comparative Example 2 |
|---|---|---|---|
| Contact Angle | 102* | 111 | 115 |
| Easiness of Washing out | Ⓞ | ○ | × |

| | | | |
|---|---|---|---|
| * The contact angle of the tablets before coating in Example 1 was determined. | | | |

As seen from Table 1, the lower the contact angle value of prepared granules to water, the easier the washing becomes.

### Comparative Example 3

7 mm φ - 9R2r core tablets (120 mg weight) were obtained according to Example 1 of Japanese Patent UN-examined Publication No. Hei 10-194969 with nateglinide H-type crystals, and then a coating solution consisting of 8 g of hydroxypropylmethylcellulose, 1.5 g of polyethylene glycol 6000, 2.4 g of talc, 0.5 g of titanium oxide and 87.6 g of purified water was sprayed onto these tablets so that 1.5 mg of hydroxypropylmetylcellulose was applied on one tablet to obtain coated tablets.

### Example 4: Evaluation of oral absorption using beagle dogs

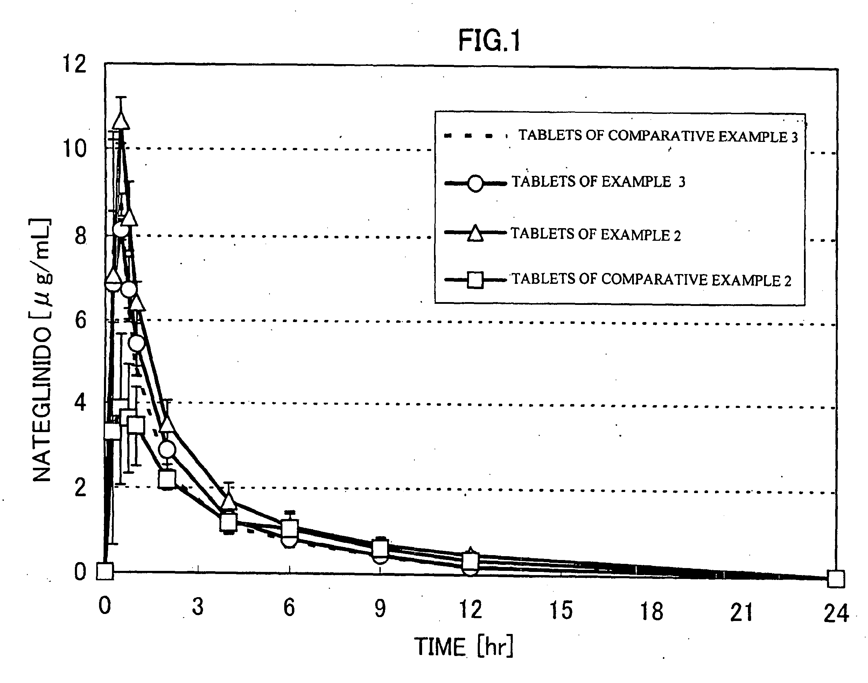

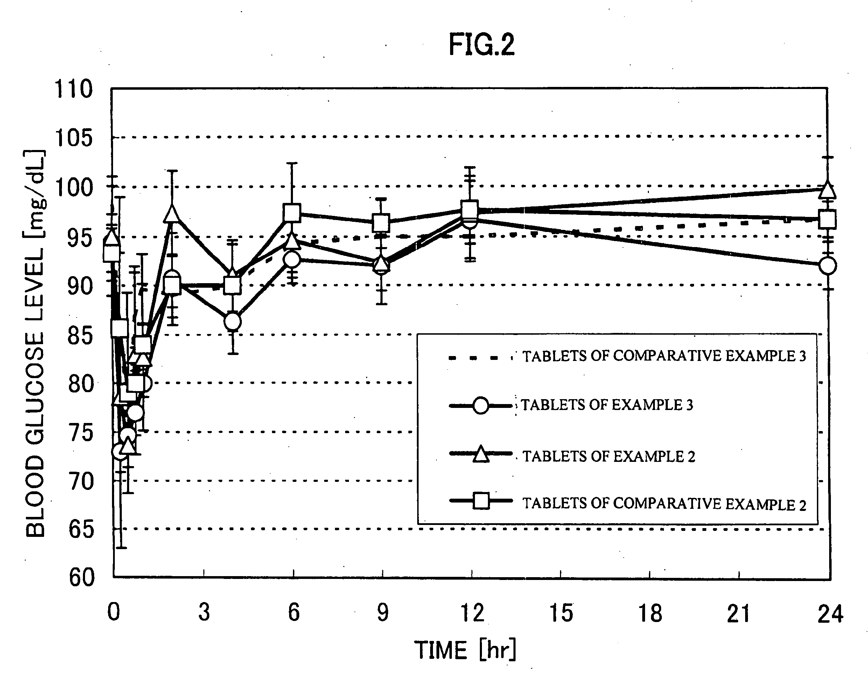

There were evaluated the profile of nateglinide concentration in plasma, the profile of blood glucose level, the pharmacokinetic parameters , and the highest down width of blood glucose level when the tablets obtained in Example.2 and 3, and Comparative Example 2 and 3 were administered to beagle dogs at 5 minutes before feeding. The results are shown in Figures 1 and 2, and Tables 2 and 3.

It was revealed that the tablets having small contact angle (tablets obtained in Example 2 and 3) exhibited Cmax, AUC, Tmax and the highest down width of blood glucose level that were equal to or higher than those of control tablets obtained in Comparative Example 3. On the other hand, decreased Cmax and prolonged Tmax were observed in the tablets having large contact angle (tablets obtained in Comparative Example 2).

**Table 2: Pharmacokinetic parameters when nateglinide tablets were administrated to beagle dogs at 5 minutes before feeding (n = 3)**

| | AUC | Cmax | Tmax |
|---|---|---|---|
| | [µg/mL•hr] | [µg/mL] | [hr] |
| Tablets of Comparative Example 3 | 18.93 | 8.76 | 0.42 |
| Tablets of Example 3 | 20.36 | 9.03 | 0.50 |
| Tablets of Example 2 | 27.55 | 11.23 | 0.42 |
| Tablets of Comparative Example 2 | 17.43 | 4.35 | 0.75 |

**Table 3: Highest down width of blood glucose level (Blood glucose level just before administration - Lowest blood glucose level) [mg/dL]**

| | Highest down width of blood glucose level [mg/dL] |
|---|---|
| Tablets of Comparative Example 3 | 23 |
| Tablets of Example 3 | 21 |
| Tablets of Example 2 | 21 |
| Tablets of Comparative Example 2 | 14 |

### Example 5: Evaluation on the storage stability of non-crystallized (amorphous) nateglinide tablets

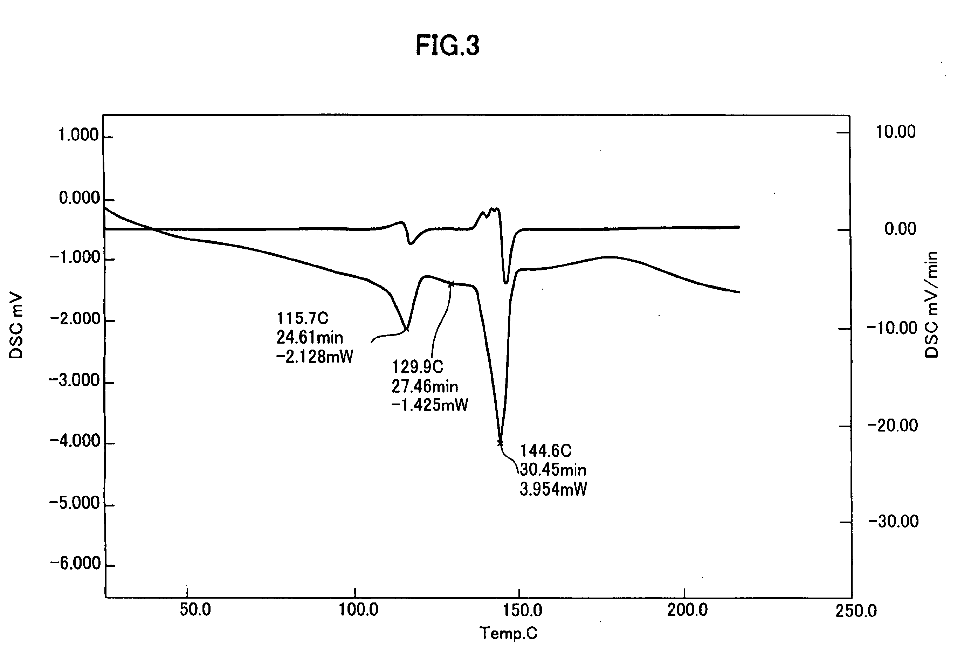

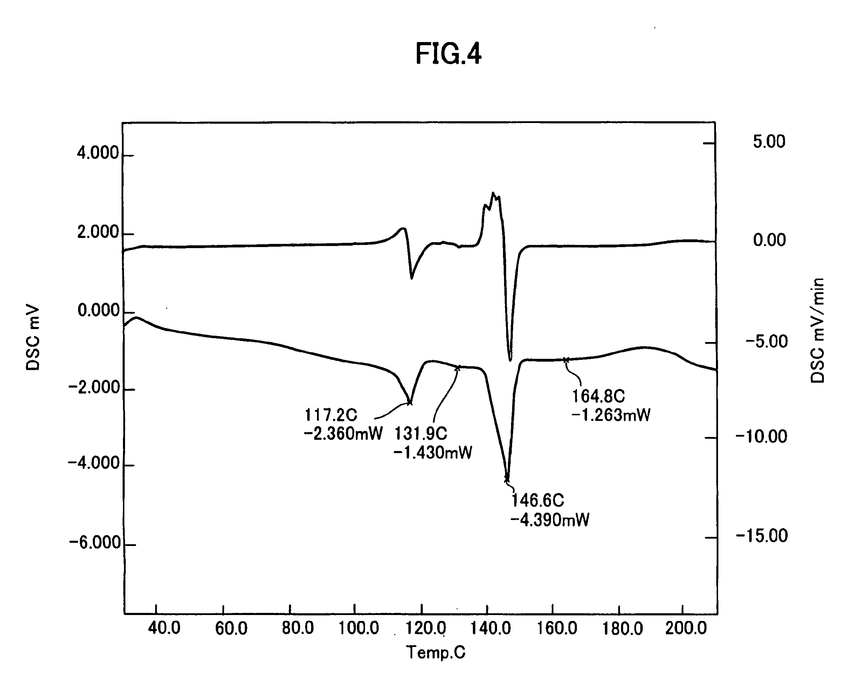

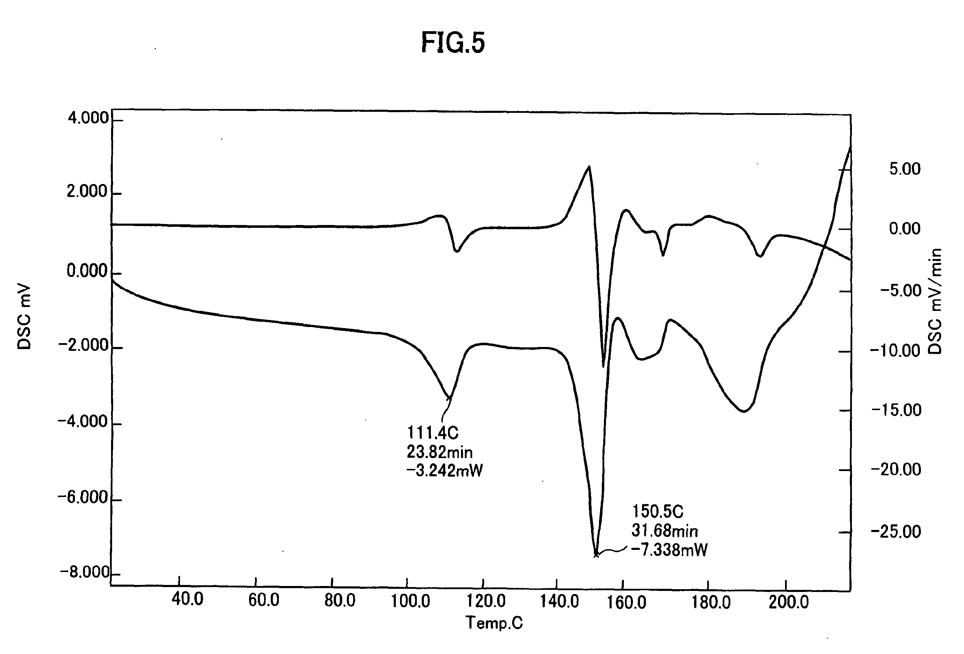

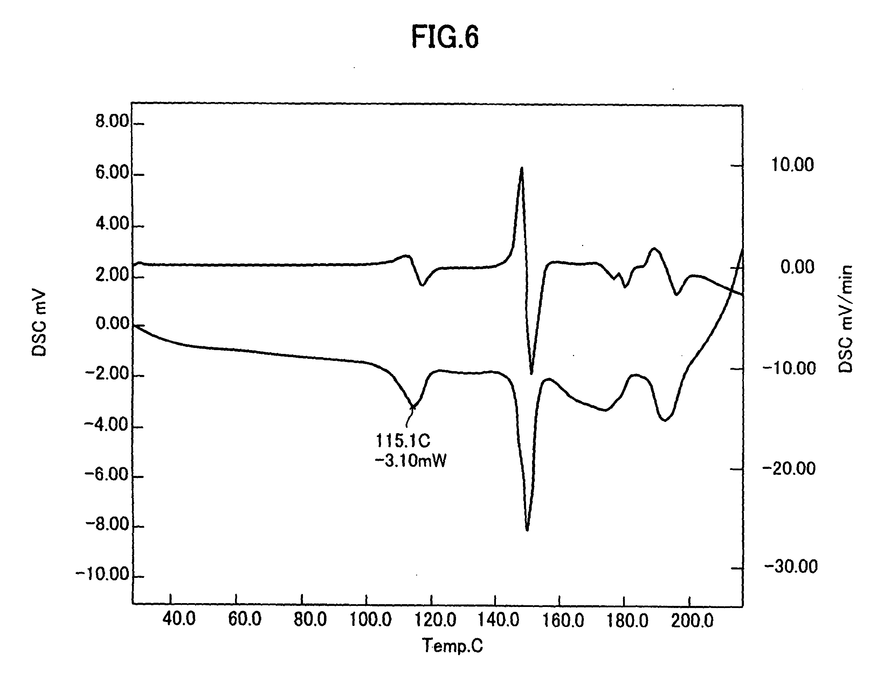

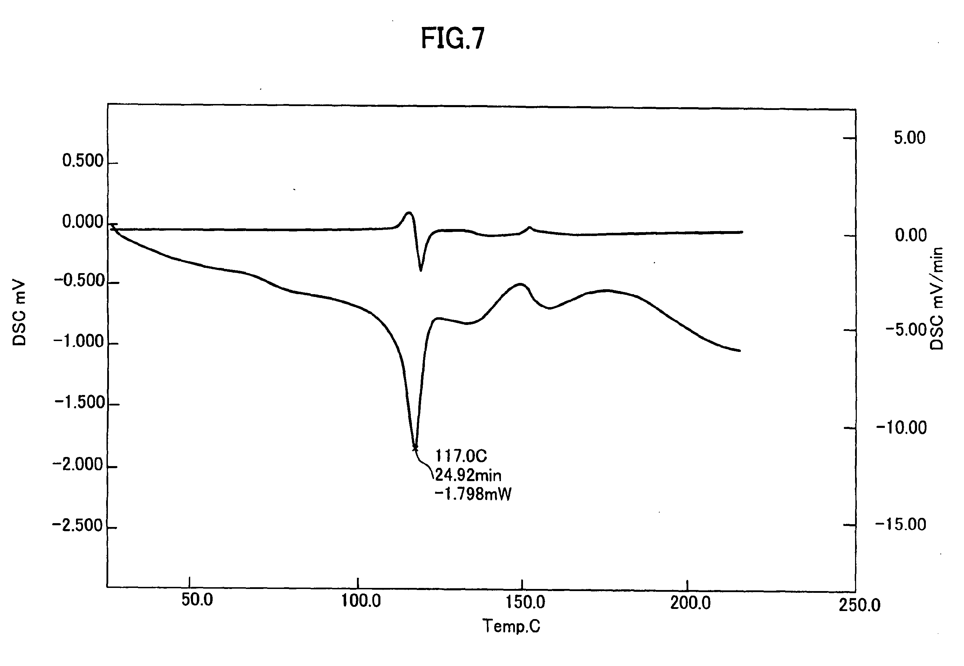

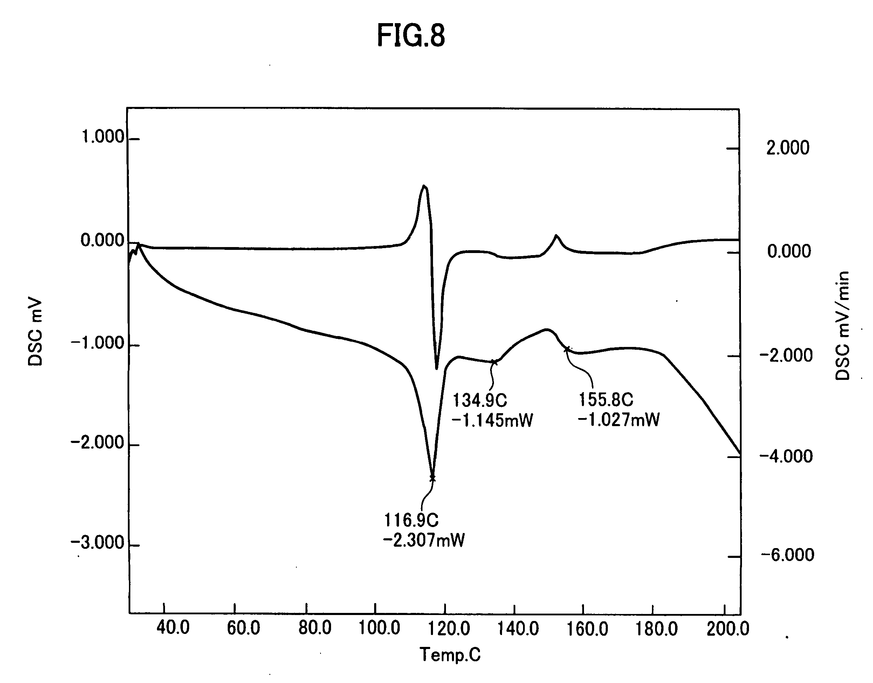

The tablets obtained according to Example 1, the tablets obtained according to Example 2 and the tablets obtained according to Example 3 were respectively packaged in aluminum pack and stored at 40°C and 75% RH for 6 months. The results evaluated for the dissolution properties in 500 ml of the solution No.2 employed in the Disintegration Test under the Japanese Pharmacopoeia using Japanese Pharmacopoeia 13th Paddle Method (50 revolutions/min; after 30 minutes) are shown in in Table 4 and DSC charts in Figure 3-8, respectively.

Before and after storing, no change was observed in both dissolution rates and DSC patterns. It can be said that 3 types of the resulting tablets are preparations having good storage stability.

**Table 4: Comparison of dissolution rates before and after storing**

| Tablets | Mean Elution Rate [%] | |
|---|---|---|
| | Initial | 40°C, 75%, 6M |
| Tablets of Example 2 | 100 | 95 |
| Tablets of Example 3 | 99 | 94 |
| Tablets of Example 1 | 100 | 100 |

According to the present invention as mentioned above, a preparation having high dissolution properties can be prepared, even when there are used nateglinide B-type crystals being able to be relatively easily prepared but being hardly soluble in water. Further, the deposition of raw composition containing nateglinide onto various apparatuses used in production of such preparation having high dissolution properties is low, and hence these apparatuses can be easily washed and reused.

## Claims

1. A hydrophilic pharmaceutical preparation containing nateglinide B-type crystals as active ingredient and one or more further components which comprise a hydrophilic substance, the contact angle of the surface of said preparation to water being 100 degrees or less.

2. A hydrophilic pharmaceutical preparation according to claim 1 which includes, in addition to the hydrophilic substance, an excipient and/or diluent, the hydrophilic substance varying the contact angle of said preparation to 100 degrees or less.

3. The hydrophilic pharmaceutical preparation according to claim 1 or claim 2,
wherein said contact angle is 90 degrees or less.

4. The hydrophilic pharmaceutical preparation according to any one of the preceding claims wherein said hydrophilic substance is selected from the groups consisting of hydrophilic polymers, surfactants, sugars, sugar alcohols and salts.

5. The hydrophilic pharmaceutical preparation according to claim 4, wherein said hydrophilic substance is a hydrophilic polymer or surfactant.

6. The hydrophilic pharmaceutical preparation according to claim 4, wherein said hydrophilic substance is a sugar alcohol.

7. The hydrophilic pharmaceutical preparation according to claim 4, wherein a combination of hydrophilic polymer and sugar alcohol is used as said hydrophilic substance.

8. The nateglinide-containing hydrophilic pharmaceutical preparation according to any one of the preceding claims which is a film coated tablet or sugar coated tablet.

9. A method for preparing a nateglinide-containing hydrophilic pharmaceutical preparation which comprises formulating such preparation by adding a hydrophilic substance(s) to formulation starting materials containing nateglinide B-type crystals as an effective ingredient, so that the contact angle of the surface of said preparation to water becomes 100 degree or less.

10. The method according to claim 9, wherein said hydrophilic substance is selected from the groups consisting of hydrophilic polymers, surfactants, sugars, sugar alcohols and salts.

11. The method according to claim 9, wherein said hydrophilic substance is a hydrophilic polymer or surfactant.

12. The method according to claim 9 wherein said hydrophilic substance is a sugar alcohol.

13. The method according to claim 9, wherein a combination of hydrophilic polymer and sugar alcohol is used as said hydrophilic substance.

14. The method according to claim 9 which further comprises applying a film or sugar to the surface of said preparation to form a film coated tablet or sugar coated tablet.

## Patentansprüche

1. Hydrophile pharmazeutische Zubereitung, die Nateglinid-Kristalle des Typs B als Wirkbestandteil und eine oder mehrere weitere Komponenten, die eine hydrophile Substanz umfassen, enthält, wobei der Kontaktwinkel der Oberfläche dieser Zubereitung gegenüber Wasser 100 Grad oder weniger beträgt.

2. Hydrophile pharmazeutische Zubereitung nach Anspruch 1, die, zusätzlich zu der hydrophilen Substanz, einen Exzipienten und/oder ein Verdünnungsmittel beinhaltet, wobei die hydrophile Substanz den Kontaktwinkel der Zubereitung auf 100 Grad oder weniger verändert.

3. Hydrophile pharmazeutische Zubereitung nach Anspruch 1 oder Anspruch 2, wobei der Kontaktwinkel 90 Grad oder weniger beträgt.

4. Hydrophile pharmazeutische Zubereitung nach einem der vorigen Ansprüche, wobei die hydrophile Substanz ausgewählt ist aus den Gruppen, bestehend aus hydrophilen Polymeren, oberflächenaktiven Substanzen, Zuckern, Zuckeralkoholen und Salzen.

5. Hydrophile pharmazeutische Zubereitung nach Anspruch 4, wobei die hydrophile Substanz ein hydrophiles Polymer oder eine oberflächenaktive Substanz ist.

6. Hydrophile pharmazeutische Zubereitung nach Anspruch 4, wobei die hydrophile Substanz ein Zuckeralkohol ist.

7. Hydrophile pharmazeutische Zubereitung nach Anspruch 4, wobei eine Kombination aus hydrophilem Polymer und Zuckeralkohol als hydrophile Substanz verwendet wird.

8. Nateglinid enthaltende hydrophile pharmazeutische Zubereitung nach einem der vorigen Ansprüche, welche eine Film-beschichtete Tablette oder eine Zucker-beschichtete Tablette ist.

9. Verfahren zur Herstellung einer Nateglinid enthaltenden hydrophilen pharmazeutischen Zubereitung, umfassend das Formulieren einer solchen Zubereitung durch Zugeben von (einer) hydrophilen Substanz(en) zu Ausgangsmaterialien der Formulierung, die Nateglinid-Kristalle des Typs B als einen Wirkbestandteil beinhalten, sodass der Kontaktwinkel der Oberfläche dieser Zubereitung gegenüber Wasser 100 Grad oder weniger wird.

10. Verfahren nach Anspruch 9, wobei die hydrophile Substanz ausgewählt ist aus den Gruppen, bestehend aus hydrophilen Polymeren, oberflächenaktiven Substanzen, Zuckern, Zuckeralkoholen und Salzen.

11. Verfahren nach Anspruch 9, wobei die hydrophile Substanz ein hydrophiles Polymer oder eine oberflächenaktive Substanz ist.

12. Verfahren nach Anspruch 9, wobei die hydrophile Substanz ein Zuckeralkohol ist.

13. Verfahren nach Anspruch 9, wobei eine Kombination aus hydrophilem Polymer und Zuckeralkohol als hydrophile Substanz verwendet wird.

14. Verfahren nach Anspruch 9, welches weiterhin umfasst, dass ein Film oder Zucker auf der Oberfläche der Zubereitung aufgebracht wird, um eine Film-beschichtete Tablette - oder eine Zucker-beschichtete Tablette auszubilden.

## Revendications

1. Préparation pharmaceutique hydrophile contenant des cristaux de natéglinide de type B comme ingrédient actif et un ou plusieurs autres composants qui comprennent une substance hydrophile, l'angle de contact de la surface de ladite préparation par rapport à l'eau étant de 100 degrés ou moins.

2. Préparation pharmaceutique hydrophile selon la revendication 1, qui comprend, en plus de la substance hydrophile, un excipient et/ou un diluant, la substance hydrophile faisant varier l'angle de contact de ladite préparation jusqu'à 100 degrés ou moins.

3. Préparation pharmaceutique hydrophile selon la revendication 1 ou la revendication 2, dans laquelle ledit angle de contact est de 90 degrés ou moins.

4. Préparation pharmaceutique hydrophile selon l'une quelconque des revendications précédentes, dans laquelle ladite substance hydrophile est choisie dans le groupe constitué des polymères hydrophiles, des tensioactifs, des sucres, des alcools de sucres et des sels.

5. Préparation pharmaceutique hydrophile selon la revendication 4, dans laquelle ladite substance hydrophile est un polymère hydrophile ou un tensioactif.

6. Préparation pharmaceutique hydrophile selon la revendication 4, dans laquelle ladite substance hydrophile est un alcool de sucre.

7. Préparation pharmaceutique hydrophile selon la revendication 4, dans laquelle une combinaison de polymère hydrophile et d'alcool de sucre est utilisée comme dite substance hydrophile.

8. Préparation pharmaceutique hydrophile contenant de la natéglinide selon l'une quelconque des revendications précédentes, qui se présente sous la forme d'un comprimé revêtu d'un film ou d'un comprimé enrobé de sucre.

9. Procédé de préparation d'une préparation pharmaceutique hydrophile contenant de la natéglinide, qui comprend la formulation de cette préparation en ajoutant une ou plusieurs substances hydrophiles à des matériaux de départ de formulation contenant des cristaux de natéglinide de type B comme ingrédient efficace, de sorte que l'angle de contact de la surface de ladite préparation par rapport à l'eau soit de 100 degrés ou moins.

10. Procédé selon la revendication 9, dans lequel ladite substance hydrophile est choisie dans le groupe constitué des polymères hydrophiles, des tensioactifs, des sucres, des alcools de sucres et des sels.

11. Procédé selon la revendication 9, dans lequel adite substance hydrophile est un polymère hydrophile ou un tensioactif.

12. Procédé selon la revendication 9, dans lequel ladite substance hydrophile est un alcool de sucre.

13. Procédé selon la revendication 9, dans lequel on utilise une combinaison de polymère hydrophile et d'alcool de sucre comme dite substance hydrophile.

14. Procédé selon la revendication 9, qui comprend en outre l'application d'un film ou de sucre à la surface de ladite préparation pour former un comprimé revêtu d'un film ou d'un comprimé enrobé de sucre.
